# EUROPÄISCHE PATENTANMELDUNG

(11) **EP 2 392 907 A1**
(43) Veröffentlichungstag der Anmeldung: **07.12.2011**
(21) Anmeldenummer: 10164589.3
(22) Anmeldetag: 01.06.2010
(51) Int. Cl.: G01N 1/10, G01N 35/00, G01N 33/18, B01L 3/00, B01L 99/00

(54) **System zur Bestimmung eines Analyten in einer Wasserprobe**

(71) Anmelder: Hach Lange GmbH, 14163 Berlin (DE)
(72) Erfinder: Zangenberg, Dietmar, 40625 Düsseldorf (DE); Schulz, Carsten, 41542 Dormagen (DE)
(74) Vertreter: Patentanwälte ter Smitten

(57) **Zusammenfassung**

Die vorliegende Erfindung betrifft ein System 10 zur Bestimmung eines Analyten in einer Wasserprobe 12. Das System 10 besteht aus mindestens einem stationären Probeentnahmeort-Identifikationssender 14, der an einem Probeentnahmeort 16 fest installiert ist, beispielsweise an einem Becken 17 einer Abwasseraufbereitungsanlage, und der drahtlos eine Probeentnahmeort-Identifikation permanent oder nach Anforderung sendet. Zum System 10 gehört ein mobiler Probenbehälter 20, der zur Probennahme der Wasserprobe 12 am Probeentnahmeort 16 geeignet ist. Der mobile Probenbehälter 20 weist eine zugeordnete Sende- und Empfangsvorrichtung 24 auf, wobei die Sende- und Empfangsvorrichtung 24 einen Speicher für die Probeentnahmeort-Identifikation und für einen Probenahme-Zeitstempel aufweist. Der Probenahme-Zeitstempel wird von einem Zeitstempel-Generator generiert. Der Zeitstempel-Generator ist mit einem Zeitstempel-Sender ausgestattet, der den Probenahme-Zeitstempel drahtlos sendet. Ferner besteht das System 10 aus einem Wasseranalysegerät 40, das die Bestimmung des Analysen in einer für die Messung aufbereiteten Wasserprobe 12 durchführt. Das Wasseranalysegerät 40 weist einen Empfänger 42 zum drahtlosen Empfangen der Probeentnahmeort-Identifikation und des Probenahme-Zeitstempels der dem Probenbehälter 20 zugeordneten Sende- und Empfangsvorrichtung 24 auf.

## Beschreibung

Die vorliegende Erfindung betrifft ein System zur Bestimmung eines Analyten in einer Wasserprobe.

Die Automatisierung und Vereinfachung von Prozessen ist unerlässlich, um eine sorgfältige und fehlerfreie Analyse von Wasserproben zu gewährleisten. In vielen Bereichen der Wasseranalytik, wie beilspielsweise in Wasseraufbereitungsanlagen, wird der gesamte Prozess der Wasseranalytik, d.h. Probennahme, Probenaufbereitung und Messung, von nicht dazu ausgebildeten Personen durchgeführt. Ferner werden die zu analysierenden Proben häufig nur unzureichend mit Informationen, beispielsweise zum Probenort, Datum und Uhrzeit, versehen. Dies kann in der Praxis zu Vertauschungen der jeweils zu einem bestimmten Zeitpunkt entnommenen Proben führen, so dass eine korrekte chronologische Aufzeichnung nicht gewährleistet werden kann. Darüberhinaus müssen die Informationen der Probe vor jeder Messung manuell in das Wasseranalysegerät eingegeben werden, was zu weiteren Fehlern und zusätzlichen Zeitverzögerungen führen kann.

Aufgabe der Erfindung demgegenüber ist es, ein System zur Bestimmung eines Anolyten in einer Wasserprobe zu schaffen, das eine einfachere und zuverlässigere Dokumentation ermöglicht.

Diese Aufgabe wird erfindungsgemäß gelöst durch ein System zur Bestimmung eines Analysen in einer Wasserprobe mit den Merkmalen des Patentanspruchs 1.

Das System zur Bestimmung eines Anolyten in einer Wasserprobe besteht aus mindestens einem stationären Probeentnahmeort-Identifikationssender, der an einem Probeentnahmeort fest installiert ist, beispielsweise an einem Becken einer Abwasseraufbereitungsanlage, und der drahtlos eine Probeentnahmeort-Identifikation permanent oder nach Anforderung sendet. Zum System gehört ein mobiler Probenbehälter, der zur Probennahme der Wasserprobe am Probeentnahmeort geeignet ist. Der mobile Probenbehälter weist eine zugeordnete Sende- und Empfangsvorrichtung auf, wobei die Sende- und Empfangsvorrichtung einen Speicher für die Probeentnahmeort-Identifikation und für einen Probenahme-Zeitstempel aufweist. Der Probenahme-Zeitstempel wird von einem Zeitstempel-Generator generiert. Der Zeitstempel-Generator ist mit einem Zeitstempel-Sender ausgestattet, der den Probenahme-Zeitstempel drahtlos sendet. Ferner besteht das System aus einem Wasseranalysegerät, das die Bestimmung des Analysen in einer für die Messung aufbereiteten Wasserprobe durchführt. Das Wasseranalysegerät weist einen Empfänger zum drahtlosen Empfangen der Probeentnahmeort-Identifikation und des Probenahme-Zeitstempels der dem Probenbehälter zugeordneten Sende- und Empfangsvorrichtung auf.

Sobald eine Wasserprobe von einem Anwender aus dem gewünschten Probeentnahmeort, beispielsweise einem Becken in einer Wasseraufbereitungsanlage, entnommen wird, empfängt die dem Probenbehälter zugeordnete Sende- und Empfangsvorrichtung drahtlos die Ortsinformation des Probeentnahmeortes, beispielsweise wenn der Probenbehälter mit der zugeordneten Sende- und Empfangsvorrichtung und der Probeentnahmeort-Identifikationssender sich innerhalb eines definierten Mindestabstandes zueinander befinden. Über den Zeitstempel-Generator erhält die Sende- und Empfangsvorrichtung eine weitere Information, nämlich eine Datum- und Zeitangabe, den sog. Probenahme-Zeitstempel. Somit wird sichergestellt, dass alle benötigten Informationen der entnommenen Wasserprobe automatisch generiert und auf einfache und fehlerfreie Weise dokumentiert werden.

Die dokumentierten Informationen der Probe werden, sobald sich der Probenbehälter mit der zugeordneten Sende- und Empfangsvorrichtung in Reichweite des Empfängers des Wasseranalysegerätes befindet, von dem Wasseranalysegerät empfangen. Somit kann jede entnommene Wasserprobe fehlerfreie und exakt zeitgestempelt zugeordnet und dokumentiert werden. Ferner gewährleistet ein derartiges System eine einfache und zuverlässige Handhabung des Analyseprozesses.

Gemäß einer bevorzugten Ausgestaltung ist der Zeitstempel-Generator ein Teil eines mobilen separaten Handgerätes, das einen Empfänger zum drahtlosen Empfangen der Probeentnahmeort-Identifikation aufweist. Die Probeentnahmeort-Identifikation kann zusammen mit dem generierten Probenahme-Zeitstempel mit Hilfe des mobilen Handgerätes an die dem Probenbehälter zugeordnete Sende- und Empfangsvorrichtung gesendet werden.

Ferner kann das mobile separate Handgerät eine Aktivierungstaste und eine Sendetaste aufweisen. Bei Betätigung der Aktivierungstaste fordert das Handgerät die Probeentnahmeort-Identifikation drahtlos vom Probeentnahmeort-Identifikationssender an und empfängt diese. Bei Betätigung der Sendetaste wird die Probeentnahmeort-Identifikation zusammen mit dem generierten Probenahme-Zeitstempel von dem Handgerät drahtlos gesendet. Darüberhinaus kann das mobile separate Handgerät einen elektrischen Energiespeicher aufweisen, das das Handgerät mit der zum Betrieb benötigten elektrischen Energie versorgt.

Mittels des mobilen separaten Handgerätes wird die Probeentnahmeort-Identifikation von dem am Probeentnahmeort fest installiertem Probeentnahmeort-Identifikationssender bei Betätigung der gegebenenfalls vorhandenen Aktivierungstaste angefordert und empfangen. Sobald das Handgerät die Probeentnahmeort-Identifikation, also die Ortsangabe, empfangen hat, wird diese Information mit dem Probenahme-Zeitstempel, also der aktuellen Datums- und Zeitangabe, ergänzt. Durch eine akustische und/oder visuelle Quittierung erhält der Anwender eine Bestätigung. Dieser Prozess wird typischerweise nach erfolgter Probennahme durchgeführt, so dass der Anwender die Informationen in einem nächstem Schritt mittels der gegebenenfalls vorhandenden Sendetaste an die Sende- und Empfangsvorrichtung des Probenbehälter überträgt, um die Dokumentation abzuschließen.

Die dokumentierten Informationen der Probe werden, sobald sich der Probenbehälter mit der zugeordneten Sende- und Empfangsvorrichtung in Reichweite des Empfängers des Wasseranalysegerätes befindet, von dem Wasseranalysegerät empfangen. Ein derartiges System gewährleistet eine einfache und zuverlässige Handhabung eines Analyseprozesses,

Alternativ kann der Zeitstempel-Generator dem Probeentnahmeort-Identifikationssender zugeordnet sein. Alternativ kann der Zeitstempel-Generator auch der Sende- und Empfangsvorrichtung des Probenbehälters zugeordnet sein. Ein dem Probeentnahmeort-Identifikationssender oder der Sende- und Empfangsvorrichtung des Probenbehälter zugeordneter Zeitstempel-Generator gewährleistet, dass alle für eine chronologische Dokumentation benötigten Informationen der entnommenen Wasserprobe automatisch innerhalb der Prozesskette dokumentiert werden.

Die Sende- und Empfangsvorrichtung ist vorzugsweise als ein wasserdichtes Etikett mit einem integrierten RFID-Chip realisiert, der an dem Probenbehälter befestigt ist. Die Sende- und Empfangsvorrichtung weist einen wiederbeschreibbaren Speicher auf, der die Orts-, Datums- und Uhrzeitangabe speichert. Das Etikett ist derart realisiert, dass eine Wiederbenutzung für mindestens 100 Spülzyklen in einer industriellen Spülmaschine gewährleistet wird.

Gemäß einer bevorzugten Ausgestaltung ist die Sende- und Empfangsvorrichtung in einem magnetischen Rührelement integriert. Dieses verbleibt während des Prozesses in dem Probenbehälter, so dass die Probennahme vom Anwender dokumentiert werden kann. Zudem bietet ein derartiges Rührelement eine Sekundärfunktion, nämlich das Homogenisieren der Probe mittels einer magnetisch angetriebenen Rührplatte.

Alternativ kann die Sende- und Empfangsvorrichtung im Probenbehälter-Körper integriert sein, beispielsweise kann die Sende- und Empfangsvorrichtung bei der Herstellung des Probenbehälters, wenn dieser im Spritzgussverfahren hergestellt wird, umspritzt oder beim Gießen eingelegt werden.

Vorzugsweise sind der Probeentnahmeort-Identifikationssender und die dem Probenbehälter zugeordnete Sende- und Empfangsvorrichtung passive Transponder. Die zum Senden und/oder Empfangen benötigte Energie wird von dem mobilen separaten Handgerät bzw. von dem Empfänger am Wasseranalysegerät als Induktionsenergie geliefert, so dass die Informationen empfangen werden können. Passive Transponder sind kosteneffizient, da ein Energiespeicher sowie zusätzliche Wartungskosten entfallen.

Vorzugsweise weist das Wasseranalysegerät eine Abruftaste auf, die bei Betätigung einen Befehl auslöst, so dass der Empfänger am Wasseranalysegerät die Probeentnahmeort-Identifikation und den Probenahme-Zeitstempel von der dem Probenbehälter zugeordneten Sende- und Empfangsvorrichtung drahtlos anfordert und empfängt.

Alternativ kann das Wasseranalysegerät die Probeentnahmeort-Identifikation und den Probenahme-Zeitstempel von der Sende- und Empfangsvorrichtung permanent und drahtlos anfordern. Sobald sich der Probenbehälter mit der zugeordneten Sende- und Empfangsvorrichtung in einer definierten Nähe zum Empfänger des Wasseranalysegerätes befindet, können die Informationen ausgelesen werden.

Vorzugsweise weist das Wasseranalysegerät eine Probenahme-Zeitstempel-Überprüfungsvorrichtung auf, mit der das Probenhalter der Wasserprobe überprüft werden kann. Die dokumentierten Informationen der entnommenen Wasserprobe werden von einem Datenverarbeitungsprogramm, welches auf einem in dem Wasseranalysegerät integrierten Computer gespeichert ist, verarbeitet und bewertet. Auf diese Weise kann der Anwender eine Information über eine Eigenschaft der Wasserprobe erhalten. Dies kann beispielsweise ein spezifisches Alter der Wasserprobe sein. Weitere dem Fachmann bekannte Applikationen, die mit einem derartigen Datenverarbeitungsprogramm möglich sind, sind ebenfalls denkbar, beispielsweise eine Warnung bei identischen Zeitstempeln mehrerer Wasserproben.

Vorzugsweise kann der Speicher der dem Probenbehälter zugeordneten Sende- und Empfangsvorrichtung durch einen Löschbefehl des mobilen separaten Handgerätes gelöscht werden. Alternativ kann der Speicher der dem Probenbehälter zugeordneten Sende- und Empfangsvorrichtung durch einen Löschbefehl des Wasseranalysegeräts gelöscht werden. Das Löschen des Speichers erfolgt beispielsweise durch Überschreiben des Speichers durch den vom Zeitstempel-Generator neu generierten Probenahme-Zeitstempel.

Nachfolgend wird ein Ausführungsbeispiel der Erfindung anhand der Zeichnung näher erläutert.

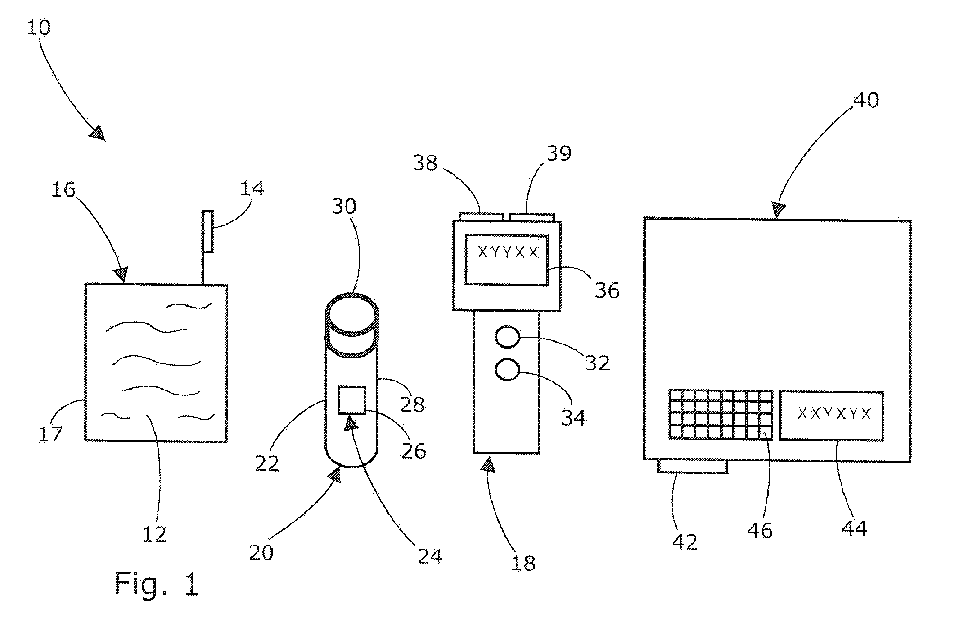

Figur 1 zeigt eine schematische Darstellung des erfindungsgemäßen Systems zur Bestimmung eines Analyten in einer Wasserprobe.

In Figur 1 ist ein System 10 zur Bestimmung eines Analyten in einer Wasserprobe 12 gezeigt. Das System 10 besteht aus mindestens einem Probeentnahmeort-Identifikationssender 14, der jeweils an einem oder mehreren Probeentnahmeorten 16 angebracht ist und der drahtlos jeweils eine individuelle Probeentnahmeort-Identiftkation nach Anforderung durch ein mobiles separates Handgerät 18 sendet.

Zum System 10 gehört ein Probenbehälter 20, der zur Probennahme der Wasserprobe 12 geeignet ist. An der äußeren Probenbehälterwand 22 ist eine dem Probenbehälter 20 zugeordnete Sende- und Empfangsvorrichtung 24 in Form eines wasserdichten RFID-Etiketts 26 angebracht. Die Sende- und Empfangsvorrichtung 24 weist einen wiederbeschreibbaren Speicher zum Speichern einer Orts-, Datums- und Zeitangabe auf. Die Sende- und Empfangsvorrichtung 24 kann alternativ im Probenbehälter-Körper 28, beispielsweise in der Probenbehälterwand 22, oder in einer Verschlusskappe 30 des Probenbehälters 20 integriert sein.

Das mobile separate Handgerät 18 weist eine Aktivierungstaste 32 auf, mit der ein Anwender (nicht gezeigt) die Probeentnahmeort-Identifikation vom Probeentnahmeort-Identifikationssender 14 anfordert, sobald eine Wasserprobe 12 aus dem Probeentnahmeort 16, beispielsweise einem Becken 17 in einer Wasseraufbereitungsanlage, entnommen wird. Bei Betätigung der Aktivierungstaste 32 sendet ein Zeitstempel-Sender 38 des mobilen separaten Handgerätes 18 eine Induktionsenergie an den Probeentnahmeort-Identifikationssender 14, der beispielsweise als ein passiver Transponder realisiert ist, so dass dieser die Probeentnahmeort-identifikation an einen Empfänger 39 des Handgerätes 18 sendet. Durch die Betätigung der Aktivierungstaste 32 wird zeitgleich ein Probenahme-Zeitstempel, d.h. eine Datums- und Zeitangabe, generiert. Alle drei Informationen werden dem Anwender auf einem Display 36 angezeigt. Ferner weist das mobile separate Handgerät 18 eine Sendetaste 34 auf, mit der der Anwender die Orts-, Datums- und Zeitangabe an die dem Probenbehälter 20 zugeordnete Sende- und Empfangsvorrichtung 24 sendet, so dass die Orts-, Datums- und Zeitangabe auf dem Speicher gespeichert wird. Bei Vorliegen einer alten Orts-, Datums- und Zeitangabe auf dem Speicher wird diese durch die von dem Handgerät 18 neu gesendete Information überschrieben.

Zum System 10 gehört ein Wasseranalysegerät 40, das einen Empfänger 42 zum drahtlosen Empfangen des auf der Sende- und Empfangsvorrichtung 24 gespeicherten Probeentnahmeort-Identifikation und des Probenahme-Zeitstempels aufweist. Der Empfänger 42 sendet permanent eine Induktionsenergie aus, so dass, sobald sich der Probenbehälter 20 mit der zugeordneten Sende- und Empfangsvorrichtung 24 in Reichweite des Empfängers 42 des Wasseranalysegerätes 40 befindet, die gespeicherten Informationen der Wasserprobe 12 empfangen und auf einem Display 44 des Wasseranalysegerätes 40 angezeigt werden. Über eine Tastatur 46 kann der Anwender weitere Eingaben zur Wasserprobe 12 am Wasseranalysegerät 40 vornehmen. Alternativ kann das Display 44 als ein Touchdisplay realisiert sein, so dass der Anwender alle möglichen Eingaben über dieses Touchdisplay vornehmen kann.

## Patentansprüche

1. System (10) zur Bestimmung eines Analyten in einer Wasserprobe (12), bestehend aus:
mindestens einem stationären Probeentnahmeort-Identifikationssender (14) an einem Probeentnahmeort (16), wobei der Probeentnahmeort-Identifikationssender (14) drahtlos eine Probeentnahmeort-Identifikation sendet,
einem mobilen Probenbehälter (20) zur Probennahme am Probeentnahmeort (16), mit einer zugeordneten Sende- und Empfangsvorrichtung (24), wobei die Sende- und Empfangsvorrichtung (24) einen Speicher für die Probeentnahmeort-Identifikation und für einen Probenahme-Zeitstempel aufweist,
einem Zeitstempel-Generator mit einem Zeitstempel-Sender, der den Probenahme-Zeitstempel drahtlos sendet, und
einem Wasseranalysegerät (40) mit einem Empfänger (42) zum drahtlosen Empfangen der Probeentnahmeort-Identifikation und des Probenahme-Zeitstempels der dem Probenbehälter (20) zugeordneten Sende- und Empfangsvorrichtung (24).

2. System (10) nach Anspruch 1, wobei der Zeitstempel-Generator Teil eines mobilen separaten Handgerätes (18) ist, das einen Empfänger (39) zum drahtlosen Empfangen der Probeentnahmeort-Identifikation von dem Probeentnahmeort-Identifikationssender (14) aufweist, wobei die Probeentnahmeort-Identifikation zusammen mit dem Probenahme-Zeitstempel von dem Zeitstempel-Sender (38) gesendet werden kann.

3. System (10) nach Anspruch 1, wobei der Zeitstempel-Generator dem Probeentnahmeort-Identifikationssender (14) am Probeentnahmeort (16) zugeordnet ist.

4. System (10) nach Anspruch 1, wobei der Zeitstempel-Generator der Sende- und Empfangsvorrichtung (24) am Probenbehälter (20) zugeordnet ist.

5. System (10) nach einem der vorangegangen Ansprüche, wobei die Sende- und Empfangsvorrichtung (24) als ein wasserdichtes Etikett (26) realisiert ist.

6. System (10) nach einem der Ansprüche 1 bis 4, wobei die Sende- und Empfangsvorrichtung (24) in einem Magnet-Rührelement in dem Probenbehälter (20) integriert ist.

7. System (10) nach einem der Ansprüche 1 bis 4, wobei die Sende- und Empfangsvorrichtung (24) im Probenbehälter-Körper (28) integriert ist.

8. System (10) nach einem der vorangegangenen Ansprüche, wobei das separate mobile Handgerät (18) eine Aktivierungstaste (32) und eine Sendetaste (34) aufweist, wobei bei Betätigung der Aktivierungstaste (32) das Handgerät (18) die Probeentnahmeort-Identifikation drahtlos anfordert und empfängt, und wobei bei Betätigung der Sendetaste (34) das Handgerät (18) die Probeentnahmeort-Identifikation und den Probenahme-Zeitstempel drahtlos sendet.

9. System (10) nach einem der vorangegangenen Ansprüche, wobei der Probeentnahmeort-Identifikationssender (14) und die dem Probenbehälter (20) zugeordnete Sende- und Empfangsvorrichtung (24) passive Transponder sind.

10. System (10) nach einem der vorangegangenen Ansprüche, wobei das Wasseranalysegerät (40) eine Abruftaste aufweist, die bei Betätigung die Probeentnahmeort-Identifikation und den Probenahme-Zeitstempel der dem Probenbehälter (20) zugeordneten Sende- und Empfangsvorrichtung (24) drahtlos anfordert und empfängt.

11. system (10) nach einem der Ansprüche 1 bis 9, wobei das Wasseranalysegerät (40) die Probeentnahmeort-Identifikation und den Probenahme-Zeitstempel der dem Probenbehälter (20) zugeordneten Sende- und Empfangsvorrichtung (24) permanent drahtlos anfordert.

12. System (10) nach einem der vorangegangenen Ansprüche, wobei das Wasseranalysegerät (40) eine Probenahme-Zeitstempel-Überprüfungsvorrichtung aufweist, mit der das Probenalter der Wasserprobe überprüft werden kann.

13. System (10) nach einem der vorangegangenen Ansprüche, wobei der Speicher der dem Probenbehälter (20) zugeordneten Sende- und Empfangsvorrichtung (24) durch einen Löschbefehl des Wasseranalysegerätes (40) gelöscht werden kann.

14. System (10) nach einem der Ansprüche 1 bis 12, wobei der Speicher der dem Probenbehälter (20) zugeordneten Sende- und Empfangsvorrichtung (24) durch einen Löschbefehl des mobilen separaten Handgerätes (18) gelöscht werden kann.
